# EUROPÄISCHE PATENTSCHRIFT

(11) **EP 3 326 695 B1**
(45) Veröffentlichungstag und Bekanntmachung des Hinweises auf die Patenterteilung: **10.06.2020**
(21) Anmeldenummer: 17192018.4
(22) Anmeldetag: 20.09.2017
(51) Int. Cl.: A61Q 5/06, A61K 8/81, A61K 8/41, A61K 8/73, A61K 8/04, A61Q 5/12, A61K 8/42

(54) **HAAR-SCHAUMFESTIGER MIT PFLEGENDER WIRKUNG**
HAIR MOUSSE WITH NOURISHING EFFECT
MOUSSE COIFFANTE POUR CHEVEUX À ACTION DE SOIN

(30) Priorität: 25.11.2016 DE 102016223395
(43) Veröffentlichungstag der Anmeldung: 30.05.2018
(73) Patentinhaber: Beiersdorf AG, 20253 Hamburg (DE)
(72) Erfinder: Demitz, Michael, 22529 Hamburg (DE); Reise, Anna, 22455 Hamburg (DE); Detert, Marion, 22455 Hamburg (DE); Sors, Nathalie, 22307 Hamburg (DE)

(56) Entgegenhaltungen:
- EP-A1- 1 632 219
- EP-A1- 1 980 293
- EP-A2- 1 319 388
- WO-A1-2009/135683
- US-A1- 2013 078 208

## Beschreibung

Die vorliegende Erfindung beschreibt eine kosmetische Zusammensetzung in Form eines Haar-Schaumfestigers mit pflegender Wirkung und gleichzeitig guten Festigungseigenschaften.

Haare sind ein wichtiger Teil des menschlichen Körpers. Der ganze menschliche Körper mit Ausnahme der Lippen, der Handinnenflächen und der Fußsohlen, ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Natürliches Haar hat in den meisten Fällen wenig eigenes Volumen und bei langem Haar hängt es oftmals recht schlaff und gerade vom Kopf herab. Viele Menschen wünschen sich aber Haare, die mehr Volumen aufweisen und sich auf gefällige Art formen (stylen) lassen. Dies kann grundsätzlich auf zweierlei Weise erzielt werden, zum einen gibt permanente Haarverformungsverfahren, beispielsweise die Dauerwelle und nicht permanente Verfahren, die die Frisur des Haares nur für eine begrenzte Zeit formen und fixieren. Um eine nichtpermanente Frisurgestaltung zu erreichen, werden mehrere Produktformen zur Verfügung gestellt, die einzeln oder in Kombination miteinander verwendet werden können. Zu nennen seien hier beispielsweise, Festiger, Schaumfestiger, Haarsprays, Haarwachse, Haargele und andere mehr.

Beim Verbraucher sehr beliebt sind Schaumfestiger, die in der Regel auf das nasse Haar aufgetragen werden. Anschließend wird das Haar zu einer Frisur geformt (gestylt), meist mithilfe eines Föns. Die Schaumfestiger zeichnen sich dadurch aus, dass sie Polymere enthalten, die eine Fixierung der Frisur bewirken und so zum Frisurerhalt geeignet sind. Zumeist werden derartige Produkte nach jeder Haarwäsche angewandt. Dies kann zur Folge haben, dass das Haar trockener und rauer wird. Konsumenten von Schaumfestigern wünschen sich daher Produkte, die neben der eigentlichen Funktion, die Frisur zu fixieren, auch die Haare pflegen können, damit die Haare weniger oder gar nicht trocken oder rau werden.

Im Stand der Technik gibt es bereits Offenbarungen zu Festigerzubereitungen, die als Schaum vorliegen.

Das Dokument WO 2010/000607 A2 offenbart Zubereitungen für das Haar, die das Haar nicht-permanent formen und als Schaum vorliegen können. Diese Zubereitungen enthalten Celluloseverbindungen und ein filmbildendes und/oder festigendes Polymer.

Im Dokument WO 2006/050788 A1 werden Haarpflegezubereitung beschrieben, die schäumend sind. Diese Zubereitungen enthalten wenigstens ein Polymer, das u.a. Haarfestigend sein kann, ein kationisches Polymer, bestimmte nichtionische und zwitterionische Tenside.

Im Dokument EP 1340485 A2 werden Aerosol-Schaumzubereitungen beschrieben, die ein oberflächenaktives Tensid, ein kationisches Polymer vom Typ Vinylpyrrolidone und eine Cellulose-haltige Komponente enthalten.

WO 2009/135683 A1 offenbart verschiedenste Haarpflegeformulierungen, Shampoos, Spülungen, Balms, Haarspitzenfluide und Haarkuren, die alle ein ausgewähltes kationisch amphoteres Polymer enthalten, synthetisierbar aus den Monomeren Vinylpyrrolidon, tert-Butylacrylat, Methacrylsäure, Dimethylaminopropylmethacrylamid und Methacryloyloxyethyldimethylethyl Ammonium Ethylsulfat.

Im Dokument EP 1319388 A2 werden Haarfestiger beschrieben, die ein Terpolymer aus Vinylpyrrolidon, Dimethylaminopropylmethacrylamid und quaternisiertem Alkyldimethylaminopropylmethacrylamid und ein nichtionisches Filmpolymer, beispielsweise Homopolymere des N-Vinylpyrrolidons und/oder Copolymere aus N-Vinylpyrrolidone und Vinylacetat enthalten.

Das Dokument EP 1980293 A1 offenbart kosmetische Zubereitungen zur Pflege des Haares, wie beispielsweise Shampos, Haarkuren, Haarspülungen, Conditioner und Haarwässer, die alle ein Parfüm und ein Copolymer enthalten, wobei das Copolymer aus DIQUAT und Acrylsäure abgeleitet ist.

Im Dokument US 2013/0078208 A1 werden quaternäre Dialkanolaminester beschrieben und der Einsatz dieser Verbindungen in Haar-kosmetischen Zubereitungen, Reinigungszubereitungen und Weichspülern für die Wäsche.

EP 1632219 A1 offenbart Zubereitungen zur Fixierung kationischer Fasern, die jeweils zwei festigende Polymere und zwei Cellulosederivate enthalten.

Ausgehend vom Stand der Technik bestand weiterhin Bedarf die Schaumfestiger zu verbessern, insbesondere im Hinblick auf die Pflegeleistung.

In der Regel geht bei Schaumfestigern mit einer Verbesserung der Pflegeleistung eine Verschlechterung der Festigungsleistung einher und umgekehrt. Der Fachmann, der Schaumfestiger entwickelt, muss immer eine ausgewogene Balance finden, damit weder die Pflegeleistung noch die Festigungswirkung so beeinträchtigt sind, dass am Ende der Anwender das Produkt nicht mehr verwenden mag.

Somit war es die Aufgabe der vorliegenden Erfindung, Haar-Schaumfestiger zu Verfügung zu stellen, die gute bis sehr gute Pflegeleistungen zeigen, ohne dass Verschlechterungen in der Festigungswirkung festzustellen sind.

Gelöst wird die vorstehende Aufgabe durch Haar-Schaumfestigerzusammensetzungen enthaltend
- ein oder mehrere festigende Polymere, wobei das/die Polymer(e) Vinylpyrrolidone-Monomere enthalten,
- einen oder mehrere Cellulosemischether,
- einen oder mehrere Esterquat(s), wobei der/die Esterquat(s) eine Propylamin-Struktureinheit aufweist/en und
- Wasser,
wobei das oder die festigenden Polymere enthaltend ein Vinylpyrrolidone Monomer ausgewählt werden aus der Gruppe Polyvinylpyrrolidone und VP/VA Coplymer.

Ebenso Gegenstand der Erfindung sind Haar-Schaumfestigerprodukte bestehend aus einer Aerosolpackungseinheit und Gesamtzusammensetzung, wobei die Gesamtzusammensetzung eine Haar-Schaumfestigerzusammensetzung und ein Treibmittel ist.

Der Wassergehalt der erfindungsgemäßen Zusammensetzungen beträgt vorteilhaft wenigstens 50 Gew.-%, bevorzugt wenigstens 55 Gew.-%, insbesondere bevorzugt 60 Gew.-%, bezogen auf das Gesamtgewicht der Haar-Schaumfestigerzusammensetzung. Der Wassergehalt der erfindungsgemäßen Zusammensetzungen beträgt vorteilhaft höchstens 90 Gew.-%, bezogen auf das Gesamtgewicht der Haar-Schaumfestigerzusammensetzung

Cellulosemischether können unter den Oberbegriff Celluloseverbindungen eingeordnet werden. Unter Celluloseverbindungen werden alle Polysaccharidverbindungen verstanden, die in ihrer Struktur Verknüpfungen von Glucoseresten enthalten, die über β-1,4-Bindungen verknüpft sind. Neben den unsubstituierten Cellulosen können die Cellulosederivate anionisch, kationisch, amphoter oder nichtionisch sein. Von diesen Derivaten sind die Celluloseether, Celluloseester und Celluloseetherester zu unterscheiden. Unter den Celluloseestern gibt es anorganische Celluloseester (Nitrate, Sulfate oder Phosphate von Cellulose ...), organische Celluloseester (Monoacetate, Triacetate, Amidopropionate, Acetatbutyrate, Acetatpropionate oder Acetattrimellitate von Cellulose ...) und gemischte organische/anorganische Ester von Cellulose, wie Acetatbutyratsulfate und Acetatpropionatsulfate von Cellulose. Von den Celluloseetherestern können die Phthalate von Hydroxypropylmethylcellulose und die Sulfate von Ethylcellulose angegeben werden.

Die erfindungsgemäßen Celluloseverbindungen sind unter den nichtionischen Celluloseethern ausgewählt. Die nichtionischen Celluloseethern umfassen Alkylcellulosen, wie beispielsweise Methylcellulosen und Ethylcellulosen; Hydroxyalkylcellulosen, wie beispielsweise Hydroxymethylcellulosen, Hydroxyethylcellulosen und Hydroxypropylcellulosen; gemischte Hydroxyalkyl-alkylcellulosen, die auch als Cellulosemischether bezeichnet werden. *Cellulosemischether* sind Verbindungen wie beispielsweise Hydroxypropylmethylcellulosen, Hydroxyethyl-methylcellulosen, Hydroxyethylethylcellulosen und Hydroxybutylmethylcellulosen.

Bevorzugt sind Hydroxypropylmethylcellulosen, geeignete Hydroxypropylmethylcellulosen können beispielsweise unter der Handelsbezeichnung Methocel 40-202 PCG bei der Dow Chemical Comp. bezogen werden.

Hydroxypropylmethylcellulosen können durch die folgende Strukturformel beschrieben werden: 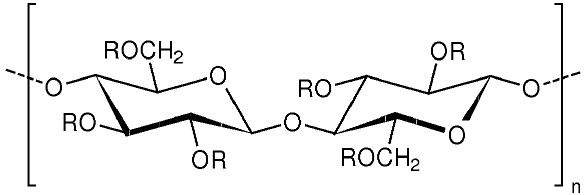 R steht für -H, -CH₃, -CH2-CHOH-CH3.

Der wenigstens eine Cellulosemischether wird in Gehalten von 0,02 Gew.-% bis 2,0. Gew.-%, bevorzugt von 0,05 Gew.-% bis 1,0 Gew.-%, insbesondere bevorzugt von 0,1 Gew.-% bis 0,5 Gew.-%, bezogen auf das Gesamtgewicht der Haar-Schaumfestigerzusammensetzung und den Aktivgehalt, eingesetzt.

Unter den geeigneten Hydroxypropylmethylcellulosen sind bevorzugt Verbindungen auszuwählen, die eine kalte Verarbeitung, d.h. eine Verarbeitung bei 15 bis 25 ºC erlauben.

Esterquats können den kationischen Tensiden zugerechnet werden. Es sind quaternisierte Verbindungen, die wenigstens eine hydrophobe Seitenkette aufweisen. Darüber hinaus sind sie in der Regel durch das Vorhandensein einer Esterbindung gekennzeichnet. Diese Esterfunktion hilft dabei, die biologische Abbaubarkeit zu verbessern. Statt der Esterfunktion kann auch eine Amidgruppe im Esterquat enthalten sein.

Vorteilhaft für die vorliegende Erfindung sind Esterquats, die eine Propylamin-Struktureinheit aufweisen. Weiter vorteilhaft sind Esterquats, die nur eine hydrophobe Seitenkette aufweisen, sogenannte Monoesterquats. Die hydrophobe Seitenkette kann 10 bis 22 Kohlenstoffatome aufweisen, bevorzugt 12 bis 20 Kohlenstoffatome, insbesondere bevorzugt 14 bis 18 Kohlenstoffatome. Ganz besonders bevorzugt ist eine Verbindung mit 16 Kohlenstoffatomen. Als Gegenionen können Halogenide, Alkylsulfat oder Alkylphosphat ausgewählt werden. Bevorzugt sind Halogenide, insbesondere bevorzugt ist Cl⁻.

Vorteilhafte Verbindungen sind beispielsweise Stearylamidopropyltrimmonium Chloride, Ricinoleamidopropyltrimonnium Chloride und Palmitamidopropyltrimonium Chloride.

Eine erfindungsgemäß bevorzugte Verbindung ist Palmitamidopropyltrimonium Chloride, das beispielsweise unter der Handelsbezeichnung Varisoft PATC von Evonik Industries bezogen werden kann.

Der wenigstens eine Esterquat, aufweisend eine Propylamin-Struktureinheit, wird in Gehalten von 0,05 Gew.-% bis 5,0 Gew.-%, bevorzugt von 0,01 Gew.-% bis 2,0 Gew.-%, insbesondere bevorzugt von 0,2 Gew.-% bis 1,0 Gew.-%, bezogen auf das Gesamtgewicht der Haar-Schaumfestigerzusammensetzung und den Aktivgehalt, eingesetzt.

Festigende Polymere bleiben nach dem Verdunsten des Lösungsmittels (oft Wasser und/oder Ethanol) als Film auf dem Haar haften und fixieren dessen Form. Für die vorliegende Erfindung werden die festigenden Polymere aus der Gruppe der Polymere, die Vinylpyrrolidone Monomere enthalten, ausgewählt, nämlich aus Polyvinylpyrrolidonen und VP/VA Coplymeren.

Homopolymere aus Vinylpyrrolidone (PVP) sind beispielsweise erhältlich als Luviskol K 30 Super P (100%) von der Firma BASF.

VP/VA Copolymere sind beispielsweise erhältlich unter der Handelsbezeichnung Luviskol VA 64 W von der Firma BASF.

Das wenigstens eine festigende Polymer, wobei das Polymer Vinylpyrrolidone-Monomere enthält, wird in Gehalten von 0,5 Gew.-% bis 20,0 Gew.-%, bevorzugt von 1,0 Gew.-% bis 10,0 Gew.-%, insbesondere bevorzugt von 1,5 Gew.-% bis 8,0 Gew.-%, bezogen auf das Gesamtgewicht der Haar-Schaumfestigerzusammensetzung und den Aktivgehalt, eingesetzt.

Die erfindungsgemäßen Schaumfestigerzusammensetzungen können weitere in der Kosmetik übliche Komponenten enthalten, mit der Maßgabe, dass sie der Erfindung nicht entgegenwirken.

So können die Haar-Schaumfestigerzusammensetzungen vorteilhaft weitere festigende Polymere enthalten. Vorteilhaft werden diese Polymere ausgewählt aus der Gruppe Acrylates/Octylacrylamide Copolymer, AMP-Acrylates/Allyl Methacrylates Copolymer, Acrylates/Hydroxyesters Acrylates Copolymer und Acrylates Copolymer.

Die weiteren festigenden Polymere liegen vorteilhaft in Gehalten von 0,2 Gew.-% bis 15,0 Gew.-%, bevorzugt von 0,5 Gew.-% bis 12,0 Gew.-%, insbesondere bevorzugt von 1,0 Gew.-% bis 10,0 Gew.-%, bezogen auf das Gesamtgewicht der Haar-Schaumfestigerzusammensetzung und den Aktivgehalt, vor.

Ebenso können die Haar-Schaumfestigerzusammensetzungen vorteilhaft ein oder mehrere kationische Polymere, insbesondere ausgewählt aus der Gruppe Polyquaternium-4, Polyquaternium-16 und Polyquaternium-68, enthalten.

Das oder die kationischen Polymere liegen vorteilhaft in Gehalten von 0,01 Gew.-% bis 10,0 Gew.-%, bevorzugt von 0,02 Gew.-% bis 5,0 Gew.-%, insbesondere bevorzugt von 0,04 Gew.-% bis 2,0 Gew.-%, bezogen auf das Gesamtgewicht der Haar-Schaumfestigerzusammensetzung und den Aktivgehalt, vor.

Verbindungen, mit denen der pH-Wert auf den gewünschten Wert eingestellt werden kann, können vorteilhaft verwendet werden. Geeignet sind alle Verbindungen, die in der kosmetischen Industrie üblich sind, bevorzugt werden jedoch die Verbindungen Citronensäure und/oder Milchsäure eingesetzt.

Weiter können zusätzlich ein oder mehrere kationische Tenside, die keine Esterquats mit einer Propylamin-Struktureinheit sind, zum Einsatz kommen. Bevorzugt werden das/die kationische(n) Tensid(e) aus der Gruppe der quartären Ammoniumsalze, insbesondere bevorzugt aus der Gruppe Cetrimonium Chlorid und Hydroxyethyl Cetyldimonium Phosphat ausgewählt. Das wenigstens eine kationische Tensid, das kein Esterquat mit einer Propylamin-Struktureinheit ist, wird in Gehalten von 0,05 Gew.-% bis 2,0 Gew.-%, bevorzugt von 0,1 Gew.-% bis 1,0 Gew.-%, bezogen auf das Gesamtgewicht der Haar-Schaumfestigerzusammensetzung und den Aktivgehalt, eingesetzt.

Ebenso können Antioxidantien erfindungsgemäß verwendet werden. Antioxidantien sind Moleküle, die verhindern, dass andere Moleküle oxidiert werden. In Oxidationsreaktionen können freie Radikale entstehen, die wiederum andere Moleküle schädigen oder zerstören. Die freien Radikale können beispielsweise durch Antioxidantien unschädlich gemacht werden. Zu den Antioxidantien können verschiedene Moleküle gezählt werden, die natürlichen oder synthetischen Ursprungs sind. Unter den synthetischen Molekülen können Verbindungen wie Butylhydroanisol (BHA), Butylhydroxytoluol (BHT) und Pentaerythrityl tetra-di-t-butyl-Hydroxyhydrocinnamat angeführt werden, erfindungsgemäß bevorzugt ist Butylhydroxytoluol und insbesondere bevorzugt ist die Verbindung Benzophenone-4.

Das wenigstens eine Antioxidans kann vorteilhaft mit einem Gehalt von 0,01 bis 1,0 Gew.-%, bevorzugt von 0,02 bis 0,7 Gew.-%, bezogen auf das Gesamtgewicht Haar-Schaumfestigerzusammensetzung und den Aktivgehalt, eingesetzt werden.

Erfindungsgemäß vorteilhaft ist ebenfalls die Verwendung von Proteinhydrolysaten, die den erfindungsgemäßen Zusammensetzungen zusätzlich zugesetzt werden können. Proteinhydrolysate sind Produktgemische, die durch sauer, basisch oder enzymatisch katalysierten Abbau von Proteinen (Eiweißen) erhalten werden. Teilhydrolysate enthalten ein Gemisch aus Peptiden unterschiedlicher Kettenlänge und gegebenenfalls auch Aminosäuren, Totalhydrolysate enthalten Gemische verschiedener Aminosäuren. Weiterhin können den Proteinhydrolysaten auch einzelne Aminosäuren und ihre Derivate sowie Gemische aus verschiedenen Aminosäuren zugerechnet werden. Die Proteinhydrolysate können sowohl pflanzlichen als auch tierischen oder marinen oder synthetischen Ursprungs sein.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan (Cognis), Promois (Interorgana), Collapuron (Cognis), Nutrilan (Cognis), Gelita-Sol (Deutsche Gelatine Fabriken Stoess & Co), Lexein (Inolex), Sericin (Pentapharm) und Kerasol (Croda) vertrieben. Auch vorteilhaft sind Proteinhydrolysate aus biogenen Fasern, wie z.B. Pashmina oder Merinowolle, und Muschelbyssos. Das jeweilige Proteinhydrolysat oder eine Kombination verschiedener Proteinhydrolysate können den erfindungsgemäßen Zusammensetzungen zusätzlich zugesetzt werden.

Das wenigstens eine Proteinhydrolysat liegt mit einem Gehalt von 0,01 bis 1,0 Gew.-%, bezogen auf das Gesamtgewicht der Haar-Schaumfestigerzusammensetzung, in den erfindungsgemäßen Zusammensetzungen vor.

Erfindungsgemäß vorteilhaft ist ebenfalls die Verwendung von Lanolinalkohol, der den erfindungsgemäßen Zusammensetzungen zusätzlich zugesetzt werden kann. Lanolinalkohol ist der unverseifbare Anteil von Wollwachs, umfassend eine Mischung aus Alkoholen mit einem mittleren Molekulargewicht von ca. 370 Da. Typische Bestandteile sind unter anderem Cholesterol, Lanosterol, Agnosterol und ihre Dihydroderivate, sowie gerade- und verzweigtkettige aliphatische Alkohole.

Ein erfindungsgemäß besonders bevorzugter Lanolinalkohol ist unter dem Handelsnamen Eucerit® bekannt. Dieser umfasst ein Gemisch aus aliphatischen Alkoholen (Alkanole mit Kettenlängen von C₁₈ bis C₂₀, Diole mit Kettenlängen von C₁₆ bis C₂₆) und Sterinen. Der Cholesterinanteil beträgt mindestens 30 %.

Lanolinalkohol ist in einem Anteil von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht der Haar-Schaumfestigerzusammensetzung, in den erfindungsgemäßen Zusammensetzungen enthalten.

Erfindungsgemäß vorteilhaft ist ebenfalls die Verwendung einer oder mehrerer Substanzen der Vitamine der B Gruppe und/oder des Vitamin B-Komplexes, die den erfindungsgemäßen Zusammensetzungen zusätzlich zugesetzt werden können. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in den erfindungsgemäßen Zusammensetzungen.

Panthenol kann beispielsweise unter dem Handelsnamen D-Panthenol 75 W als 77% Lösung in Wasser von der BASF bezogen werden.

Eine oder mehrere Substanzen der Vitamine der B Gruppe und/oder des Vitamin B-Komplex sind erfindungsgemäß in einem Anteil von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht der Haar-Schaumfestigerzusammensetzung und den Aktivgehalt, in den erfindungsgemäßen Zusammensetzungen enthalten.

Erfindungsgemäß vorteilhaft ist ebenfalls die Verwendung von Pflanzenextrakten und/oder Pflanzenölen, wobei die Pflanzenextrakte sowohl wasserlöslich als auch öllöslich sein können. Vorteilhaft sind wässrige, Glycol-haltige Extrakte und wässrige, Glycerin-haltige Extrakte, wobei die wässrigen, Glycerin-haltigen Extrakte bevorzugt sind. Insbesondere vorteilhaft ist ein wässriger, Glycerin-haltiger Extrakt von Macadamia (*Macadamia integrifolia*). Ein derartiger Extrakt ist unter dem Handelsnamen Extrapone Macadamia Nut GW CL® bekannt.

Die Pflanzenextrakte oder Pflanzenöl können einzeln oder in Kombination eingesetzt werden. Die Gesamtmenge an Pflanzenextrakt(en) und/oder Pflanzenöl(en) beträgt 0,005 Gew.-% bis 5 Gew.-%, bevorzugt 0,05 Gew.-% bis 2,0 Gew.-%, insbesondere bevorzugt 0,01 Gew.-% bis 1,0 Gew.-%, bezogen auf das Gesamtgewicht der Haar-Schaumfestigerzusammensetzung.

Die erfindungsgemäßen Zusammensetzungen können konserviert werden. Dazu können alle Konservierungsstoffe verwendet werden, die gemäß Kosmetikverordnung verwendet werden dürfen. Bevorzugt ist jedoch eine Konservierung mit Benzoesäure und/oder ihren Salzen.

Die jeweiligen Konservierungsstoffe können einzeln oder in Kombination eingesetzt werden. Die Gesamtmenge an einem Konservierungsstoff oder mehreren Konservierungsstoffen wird vorteilhaft gewählt aus dem Bereich von 0,01 bis 1,0 Gew.-%, bevorzugt 0,1 bis 0,8 Gew.-%, bezogen auf das Gesamtgewicht der Haar-Schaumfestigerzusammensetzung und den Aktivgehalt.

Das Schaumfestigerprodukt umfasst eine Verpackungseinheit und eine Gesamtzusammensetzung, die vorteilhaft wiederum aus der Schaumfestigerzusammensetzung und einem Treibmittel besteht.

Die Verpackungseinheit ist erfindungsgemäß vorteilhaft ein Aerosolbehälter mit Sprühvorrichtungen. Die Aerosolbehälter enthalten die Schaumfestigerzusammensetzung und das Treibmittel. Aerosolbehälter stehen unter Druck des Treibmittels. Derartige Behälter können mit Ventilen sehr unterschiedlicher Bauart ausgestattet sein, die ein Aufschäumen bei der Entnahme des Inhalts ermöglichen.

Als Druckgasbehälter kommen im Sinne der vorliegenden Erfindung vor allem zylindrische Gefäße aus Metall (Aluminium, Weißblech, Inhalt <1000 ml) in Frage, bei deren Auswahl Druck- und Bruchfestigkeit, Korrosionsbeständigkeit, leichte Füllbarkeit, usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens.

Besonders vorteilhaft im Sinne der vorliegenden Erfindung sind Dosen aus Weißblech oder Aluminium. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- sowie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt.

Der innere Aufbau der Sprüh-Dosen sowie die Ventilkonstruktion sind je nach VerwendungsZweck und der physikalischen Beschaffenheit des Inhalts - z. B. ob als Zwei- oder als Dreiphasensystem - sehr variantenreich und können vom Fachmann durch einfaches Ausprobieren ohne erfinderisches Zutun ermittelt werden. Für geeignete Ausführungsformen sei auf das "Aerosol Technologie Handbuch der Aersosol-Verpackung" hingewiesen (Wolfgang Tauscher, Melcher Verlag GmbH HeidelberglMünchen, 1996*).*

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:

| | |
|---|---|
| Teller: | Weißblech: blank, gold- bzw. klarlackiert, folienkaschiert (PE, PP oder PET) Aluminium: blank, silber- oder goldlackiert, verschiedene Lackvarianten, Stoner-Mudge-Ausführung. |
| Dichtung: | natürliche bzw. synthetische Elastomere bzw. thermoplastische (Sleeve-Gaskets, folienkaschiert aus PE oder PP) Innen- und Außendichtungen, z. B. aus Perbunan, Buna, Neopren, Butyl, CLB, LDPE, Viton, EPDM, Chlorbutyl, Brombutyl und/oder diversen Compounds. |
| Kegel: | Polyamid (PA), Polyoxymethylen (POM), Messing sowie diversen Sondermaterialen, Standardbohrungen (z. B.: 0,25 bis 0,70 mm oder 2 x 0,45 bis 2 x 1,00 mm), verschiedene Schaftdurchmesser |
| Feder: | Metall, besonders bevorzugt V2A, rostfreier Stahl; Kunststoff und auch Elastomer. |
| Gehäuse: | Standard und Impact |
| | VPH-Bohrungen, RPT-Bohrungen oder geschlitzt für Überkopf-Anwendungen Materialien: z. B. Polyacetal, PA, PE, POM und dergleichen mehr |
| Steigrohr: | Kunststoff (Polymer Resin), z.B. PE, PP, PA oder Polycarbonat. |

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Es ist erfindungsgemäß bevorzugt, wenn der Aerosolbehälter eine Aluminiumdose ist, die auf der Innenseite mit einem Schutzlack, insbesondere mit Epoxyphenol-Lack, beschichtet ist.

Erfindungsgemäß können die Treibgase des Treibmittels vorteilhaft ausgewählt werden aus Kohlenwasserstoffgasen, fluorierten Gasen, Fluorkohlenwasserstoffgase, Dimethylether, Stickstoff, Luft oder Kohlendioxid oder Mischungen davon. Bevorzugt sind Treibmittel, die eine Mischung aus den Treibgasen Isobutan, Propan und n-Butan sind.

Die bevorzugte Druckgasstufe wird vorteilhaft aus dem Bereich von 2,7 bis 3,5 bar ausgewählt.

Vorteilhaft im Sinne der vorliegenden Erfindung wird der Gewichtsanteil an Treibmittel aus dem Bereich von 15,0 bis 1,0 Gew.-%, insbesondere 12,0 bis 2,0 Gew.-%, bezogen auf das Gesamtgewicht der Gesamtzusammensetzung, die aus Schaumfestigerzusammensetzung und Treibmittel besteht, gewählt. Die Schaumfestigerzusammensetzung liegt demnach mit einem Gewichtsanteil von 99 bis 85 Gew.-%, insbesondere 98 bis 88 Gew.-% in der Gesamtzusammensetzung vor.

Die erfindungsgemäßen Schaumfestigerzusammensetzungen liegen vorteilhaft in Form von verschäumbaren oder nachschäumbaren Zusammensetzungen vor, welche aus Aerosolbehältern entnommen werden und beim Austritt aus der Düse aufschäumen. Erfindungsgemäß vorteilhafte Aerosolbehälter sind Sprühvorrichtungen mit einer Füllung aus einer weitgehend flüssigen Schaumfestigerzusammensetzung, die unter dem Druck eines Treibmittels stehen. Derartige Behälter können mit Ventilen sehr unterschiedlicher Bauart ausgestattet sein, die ein Aufschäumen bei der Entnahme des Inhalts ermöglichen.

Eine Kombination aus einem oder mehreren festigenden Polymeren, die Vinylpyrrolidone-Monomere enthalten, einem oder mehreren Cellulosemischethern und einem oder mehreren Esterquat(s), die eine Propylamin-Struktureinheit aufweisen, führt zu verbesserten Eigenschaften wie Gleitvermögen, Haarstruktur und Geschmeidigkeit im trockenen Haar.

Es wurde auch festgestellt, dass das Haar weniger aufgeladen, weniger rauh und weniger klebrig ist. Die Festigungswirkung, ausgedrückt als Haltestufe, wird dabei überraschenderweise nicht beeinträchtigt.

Dies gilt insbesondere für eine Ausführungsform enthaltend als festigendes Polymer ein Vinylpyrrolidone (VP)/Vinylacetat (VA) Coplymer, als Cellulosmischether Hydroxypropyl Methylcellulose und als Esterquat einen Monoesterquat. Besonders vorteilhaft bezüglich der oben genannten Eigenschaften im trockenen Haar ist eine Ausführungsform, die als festigendes Polymer ein Vinylpyrrolidone (VP)/Vinylacetat (VA) Coplymer, als Cellulosmischether Hydroxypropyl Methylcellulose und als Esterquat Palmitamidopropyltrimonium Chloride (PATC) enthält.

Veranschaulicht werden diese Effekte durch die nachfolgend aufgeführten Untersuchungen. Die nachfolgenden Rezepturbeispiele geben die Konzentrationen der eingesetzten Rohstoffe an, nicht die Aktivgehalte, jeweils in Gewichtsprozent, sofern keine anderweitigen Angaben gemacht werden.

| **Rohstoff** | **Haar-Schaumfestigerzusammensetzung Beispielrezeptur** | | |
|---|---|---|---|
| | 1 | **2** | 3 |
| D-Panthenol 75 W, BASF | 0,1 | 0,1 | 0,1 |
| Varisoft PATC | 0,5 | 0,0 | 0,5 |
| Eumulgin CO 40 | 0,3 | 0,3 | 0,3 |
| Methocel 40-202 PCG | 0,2 | 0,2 | 0,0 |
| Luviquat Supreme AT 1 | 3,5 | 3,5 | 3,5 |
| Luviskol VA 64 W | 6,7 | 6,7 | 6,7 |
| Parfum | 0,2 | 0,2 | 0,2 |
| Citronensäure Monohydrat | 0,1 | 0,1 | 0,1 |
| Natriumbenzoat Pharma | 0,2 | 0,2 | 0,2 |
| Uvinul MS 40 | 0,1 | 0,1 | 0,1 |
| Aqua | Ad 100 | Ad 100 | Ad 100 |

| | **Haar-Schaumfestigerzusammensetzung** | **1** | **2** | **3** |
|---|---|---|---|---|
| | Menge | 91,0 | 91,0 | 91,0 |
| 17 | **Treibmittel:** Propan/Butan/Isobutan 3,0 bar | 9,0 | 9,0 | 9,0 |

| **Rohstoff/Handelsname** | **INCI-Bezeichnung** |
|---|---|
| D-Panthenol 75 W, BASF (77 % aktiv) | Panthenol |
| Varisoft PATC (60 % aktiv) | Palmitamidopropyltrimonium Chloride |
| Eumulgin CO 40 (100% aktiv) | PEG-40 Hydrogenated Castor Oil |
| Luviquat Supreme AT 1 (20 % aktiv) | Polyquaternium-68 |
| Luviskol VA 64 W (50% aktiv) | VP/VA Copolymer |
| Methocel 40-202 PCG (99% aktiv) | Hydroxypropyl Methylcellulose |
| Citronensäure Monohydrat (100 5 aktiv) | Citric Acid |
| Natriumbenzoat Pharma (100 % aktiv) | Sodium Benzoate |
| Uvinul MS 40 (100 % aktiv) | Benzophenone-4 |

In einem ersten Schritt wurden die Pflegeeigenschaften erfindungsgemäßer Schaumfestigerzusammensetzungen *in vitro* analysiert. Dazu wurden die Schaumfestiger gemäß den Rezepturbeispielen 1 bis 3 auf nasse, ungebleichte Tressen aufgetragen und über Nacht bei Raumtemperatur getrocknet. Anschließend beurteilte ein Expertenpanel (n=4) die Pflegeleistung und Sensorik der Produkte am Haar. Die Parameter Gleitvermögen und Haarstruktur wurden anhand einer 10er-Skala mit 1 (schlechteste Bewertung) bis 10 (bestmögliche Bewertung) bewertet. Dargestellt sind die Mittelwerte (MW) aus den Expertenbewertungen.

| **Beispielrezeptur** | **1** | **2** | **3** |
|---|---|---|---|
| Gleitvermögen (MW) | 8 | 6 | 7,75 |
| Haarstruktur (MW) | 7,25 | 5 | 6,75 |

Die Parameter geschmeidig, aufgeladen, rau und klebrig zeigen an, wie viele der Panellisten diese Eigenschaften wahrgenommen haben (0 ist somit die bestmögliche Bewertung, 4 die schlechteste Bewertung).

| **Beispielrezeptur** | **1** | **2** | **3** |
|---|---|---|---|
| geschmeidig | 3 | 0 | 2 |
| aufgeladen | 0 | 3 | 0 |
| rau | 0 | 3 | 0 |
| klebrig | 0 | 1 | 0 |

Die Anwendung eines Produktes gemäß Beispielrezeptur 1 (enthaltend Hydroxypropyl Methylcellulose und PATC) führt zu deutlich besseren Bewertungen in den sechs untersuchten Pflegeeigenschaften im trockenen Haar im Vergleich zu den Bewertungen die nach Verwendung von Produkten gemäß den Beispielrezepturen 2 (nur Hydroxypropyl Methylcellulose) und Beispielrezeptur 3 (nur PATC) erzielt werden.

Im nächsten Schritt wurde die Festigungswirkung, ausgedrückt als Haltestufe, *in vivo* untersucht. Es ist allgemein bekannt, dass eine Verbesserung der Pflegeeigenschaften zumeist die haltgebenden Stylingeigenschaften verschlechtert. Überraschenderweise zeigte sich jedoch, dass die Anwendung von Produkten gemäß Beispielrezeptur 1 in Bezug auf die Halteperformance zu vergleichbaren Ergebnissen führt wie die Anwendung eines käuflich zu erwerbenden Produktes, nämlich NIVEA Schaumfestiger extra stark.

Dazu wurden semi-monadische Home-in-Use-Anwendungsstudien in Deutschland mit verblendeten Packmitteln und regelmäßigen Schaumfestigerverwenderinnen (über 90 Testerinnen) durchgeführt. Die Bewertung erfolgte anhand einer 7er-Skala nach der Anwendung, wobei .1 kaum Halt, also eine schlechte Bewertung bedeutet und 7 viel Halt, also eine gute Bewertung bedeutet. Verglichen wurde ein Produkt, das bereits käuflich zu erwerben ist und einen extra starken Halt ausweist mit einem Produkt gemäß Beispiel Rezeptur 1.

Das käuflich zu erwerbende Produkt ist ein Nivea Schaumfestiger extra stark. Die INCI-Liste dieses Produktes zeigt folgende Inhaltsstoffe an:
Aqua, Isobutane, VP/VA Copolymer, Propane, Macadamia Integrifolia Seed Oil, Panthenol, Niacinamide, Benzophenone-4, Polyquaternium-68, Polyquaternium-4, Cetrimonium Chloride, Hydroxyethyl Cetyldimonium Phosphate, Butane, PEG-40 Hydrogenated Castor Oil, Glycerin, Citric Acid, Sodium Benzoate, Phenoxyethanol, Ethylhexylglycerin, Linalool, Citronellol, Geraniol, Benzyl Alcohol, Butylphenyl Methylpropional, Limonene, Amyl Cinnamal, Alpha-Isomethyl lonone, Parfum.

| | **NIVEA Schaumfestiger extra stark** | **Produkt gemäß Beispielrezeptur** 1 |
|---|---|---|
| The product gives the hair extra strong hold; das Produkt gibt dem Haar einen extra-starken Halt. | 5,5 | 5,7 |
| The product gives the hairstyle stability for a long time; das Produkt gibt der Frisur Stabilität für eine lange Zeit. | 5,7 | 5,8 |

Hier zeigt sich, dass das erfindungsgemäße Produkt sogar etwas besser bewertet wird, als ein bereits käuflich zu erwerbendes Produkt, das für einen extra-starken Halt vorgesehen ist.

### Beispiele

Nachfolgende Beispiele sollen die Erfindung verdeutlichen, ohne sie einzuschränken. Dabei geben die angeführten Mengen die Konzentrationen der Rohstoffe an, nicht den Aktivgehalt, jeweils in Gewichtsprozent, sofern keine anderen Angaben gemacht wurden.

| | | **4** | **5** | **6** | **7** | **8** | **9** | **10** | **11** | **12** |
|---|---|---|---|---|---|---|---|---|---|---|
| 1 | Sorbitol | - | - | 2,6 | 2,6 | - | 2,6 | - | 1,0 | 8,2 |
| 2 | PVP/VA Copolymer | 5,7 | 5,7 | 5,7 | 5,4 | 6,3 | 5,7 | 7,6 | 5,4 | 12 |
| 3 | Polyquaternium-68 | 3,9 | 3,9 | 3,9 | 1,4 | 4,5 | 3,9 | - | - | 5 |
| 4 | Palmitamidopropyltrimonium Chloride | 0,5 | 0,5 | 0,4 | 0,5 | 0,6 | 0,8 | 0,7 | 0,5 | 0,5 |
| 5 | Hydroxypropyl Methylcellulose | 0,2 | 0,2 | 0,1 | 0,3 | 0,2 | 0,2 | 0,4 | 0,2 | 0,2 |
| 6 | Panthenol | 0,1 | 0,1 | 0,1 | 0,1 | 0,1 | 0,1 | 0,1 | - | 0,3 |
| 7 | Niacinamide | 0,02 | 0,02 | 0,02 | 0,02 | 0,02 | 0,02 | 0,02 | - | 0,1 |
| 8 | Citronensäure | 0,03 | 0,03 | 0,03 | 0,03 | 0,09 | 0,03 | 0,02 | 0,02 | 0,3 |
| 9 | Natriumbenzoat | 0,2 | 0,2 | 0,2 | 0,2 | 0,2 | 0,2 | 0,2 | 0,18 | 0,1 |
| 10 | Laureth-3 | - | - | - | - | - | - | - | 0,5 | 0,8 |
| 11 | PEG-8 | 1,8 | - | - | - | - | - | 0,9 | - | - |
| 12 | Benzophenone-4 | 0,05 | 0,05 | 0,05 | - | - | - | - | - | - |
| 13 | Trideceth-12 | - | - | - | - | - | - | - | 0,5 | 0,8 |
| 14 | PEG-40 Hydrogenated Castor Oil | 0,3 | 0,3 | 0,3 | 0,3 | 0,3 | 0,3 | 0,3 | 0,2 | 0,2 |
| 15 | Ceteareth-20 | - | - | - | - | - | - | 0,18 | - | 0,09 |
| 16 | Parfüm | q.s. | q.s. | q.s. | q.s. | q.s. | q.s. | q.s. | q.s. | q.s. |
| 31 | Lanolin Alcohol | | 0,1 | | | | | 0,05 | | |
| 32 | Macadamia Integrifolia Seed Oil | | | 0,1 | | | | | | |
| | Wasser | ad 100 | ad 100 | ad 100 | ad 100 | ad 100 | ad 100 | ad 100 | ad 100 | ad 100 |
| | | | | | | | | | | |
| | Haar-Schaumfestigerzusammensetzung | 91 | 90 | 89 | 90 | 91 | 91 | 91 | 90 | 88 |
| 17 | Propan/Butan/Isobutan 3,0 bar | 9 | 10 | 11 | 10 | 9 | 9 | 9 | 10 | 12 |

### Weitere Beispiele:

| | | **13** | **14** |
|---|---|---|---|
| 1 | Sorbitol | 5,5 | 3,7 |
| 2 | PVP/VA Copolymer | 12,2 | 11,7 |
| 3 | Polyquaternium-68 | 5,4 | - |
| 18 | PVP | - | 3,3 |
| 4 | Palmitamidopropyltrimonium Chloride I | 0,5 | 0,5 |
| 5 | Hydroxypropyl Methylcellulose | 0,2 | ,02 |
| 6 | Panthenol | 0,05 | 0,3 |
| 7 | Niacinamide | 0,04 | 0,09 |
| 8 | Citronensäure | 0,23 | 0,21 |
| 9 | Natriumbenzoat | 0,20 | 0,20 |
| 10 | Laureth-3 | - | 0,8 |
| 19 | Laureth-4 | 0,8 | - |
| 13 | Trideceth-12 | 0,6 | 0,8 |
| 14 | PEG-40 Hydrogenated Castor Oil | 0,2 | 0,3 |
| | Parfüm | q.s. | q.s. |
| | Wasser | Ad 100 | Ad 100 |
| | | | |
| | Haar-Schaumfestigerzusammensetzung | 91 | 91 |
| 17 | Propan/Butan/Isobutan 3,0 bar | 9 | 9 |

### Weitere Beispiele:

| | | **15** | **16** |
|---|---|---|---|
| 1 | Sorbitol | 3,4 | 5,6 |
| 2 | PVP/VA Copolymer | 3,9 | 2,2 |
| 20 | Polyquaternium-16 | 7,8 | 5,3 |
| 33 | Polyquarterium-4 | 0,05 | 0,1 |
| 4 | Palmitamidopropyltrimonium Chloride | 0,6 | 0,8 |
| 5 | Hydroxypropyl Methylcellulose | 0,3 | 0,2 |
| 6 | Panthenol | 0,15 | 0,05 |
| 7 | Niacinamide | - | 0,02 |
| 8 | Citronensäure | 0,31 | 0,17 |
| 9 | Natriumbenzoat | 0,2 | - |
| 10 | Laureth-3 | 0,5 | 0,5 |
| 13 | Trideceth-12 | 0,5 | 0,5 |
| 14 | PEG-40 Hydrogenated Castor Oil | 0,2 | 0,2 |
| 16 | Parfüm | q.s. | q.s. |
| | Wasser | ad 100 | ad 100 |
| 21 | Ethanol | - | 15,0 |
| | | | |
| 17 | Haar-Schaumfestigerzusammensetzung | 90 | 90 |
| | Propan/Butan/Isobutan 3,0 bar | 10 | 10 |

### Weitere Beispiele:

| | | **14** | **15** | **16** | **17** |
|---|---|---|---|---|---|
| 1 | Sorbitol | 1,4 | 3,7 | 5,3 | 8,1 |
| 2 | PVP/VA Copolymer | 8,7 | 4,9 | 3,0 | 2,5 |
| 23 | AMP-Acrylates/Allyl Methacrylates Copolymer | 6,3 | - | - | - |
| 24 | Acrylates/Hydroxyesters Acrylates Copolymer | - | - | 10,0 | 9,9 |
| 25 | Acrylates Copolymer | - | 4,2 | - | - |
| 22 | Acrylates/Octylacrylamide Copolymer | - | - | - | 5,5 |
| 3 | Polyquaternium-68 | 3,2 | 5,0 | - | - |
| 20 | Polyquaternium-16 | 0,5 | - | - | - |
| 26 | Cetrimonium Chloride | | | 0,6 | |
| 27 | Hydroxyethyl Cetyldimonium Phosphate | | 0,4 | | |
| 28 | Aminomethylpropanol | - | 0,1 | 0,1 | 0,1 |
| 4 | Palmitamidopropyltrimonium Chloride | 0,5 | 0,5 | 0,8 | 0,7 |
| 5 | Hydroxypropyl Methylcellulose | 0,3 | 0,2- | 0,3 | 0,4 |
| 6 | Panthenol | 0,1 | 0,1 | 0,1 | 0,1 |
| 29 | Phenoxyethanol | 0,3 | 0,5 | 0,8 | 0,5 |
| 30 | Parabene | 0,5 | 0,2 | 0,2 | 0,8 |
| 14 | PEG-40 Hydrogenated Castor Oil | 0,2 | 0,4 | - | 0,2 |
| | Parfüm | q.s. | q.s. | q.s. | q.s. |
| | Wasser | Ad 100 | Ad 100 | Ad 100 | Ad 100 |
| | Haar-Schaumfestigerzusammensetzung | 90 | 90 | 90 | 90 |
| 17 | Propan/Butan/Isobutan 3,0 bar | 10 | 10 | 10 | 10 |

### Übersicht INCI-Bezeichnunaen und Rohstoffnamen:

| | | |
|---|---|---|
| 1 | Sorbitol | C Sorbidex NC 16225 von Cargill, 70% aktiv |
| 2 | PVP/VA Copolymer | Luviskol VA 64 W von BASF, 50% aktiv |
| 3 | Polyquaternium-68 | Luviquat Supreme® von BASF, 20% aktiv |
| 4 | Palmitamidopropyltrimonium Chloride | Varisoft PATC, Firma Evonik, 60%aktiv |
| 5 | Hydroxypropyl Methylcellulose | Methocel 40-202 PCG, 99% aktiv |
| 6 | Panthenol | D-Panthenol 75 W, BASF, 77% aktiv |
| 7 | Niacinamide | Niacinamide, Lonza, 99% aktiv |
| 8 | Citronensäure | Citronensäure Monohydrat |
| 9 | Natriumbenzoat | Natriumbenzoat Pharma |
| 10 | Laureth-3 | Dehydol LS3 Deo N® von Cognis, 1 00% aktiv |
| 11 | PEG-8 | Polyglykol 400 von Clariant, 99% aktiv |
| 12 | Benzophenone-4 | Uvinul MS 40, 100% aktiv |
| 13 | Trideceth-12 | LIPOCOL L-12, Induchem, 1 00% aktiv |
| 14 | PEG-40 Hydrogenated Castor Oil | Eumulgin CO 40, 1 00% aktiv |
| 15 | Ceteareth-20 | Eumulgin B20® von Cognis |
| 16 | Parfüm | |
| 17 | Propan/Butan/Isobutan 3,0 bar | Drivosol 35 A von Evonik |
| 18 | PVP | Luviskol K 30 Pulver von BASF |
| 19 | Laureth-4 | Brij 30® von Croda, 1 00% aktiv |
| 20 | Polyquaternium-16 | Luviquat FC 370 (BASF), 40% aktiv |
| 21 | Ethanol | Agraralkohol Surfin Sorte 616 von France Alcools, 96, 12% aktiv |
| 22 | Acrylates/Octylacrylamide Copolymer | Amphomer® von AkzoNobel, 97% aktiv |
| 23 | AMP-Acrylates/Allyl Methacrylates Copolymer | FIXATE G-100, Lubrizol, 26% aktiv |
| 24 | Acrylates/Hydroxyesters Acrylates Copolymer | Acudyne 180, Dow, 58% aktiv |
| 25 | Acrylates Copolymer | Avalure AC 115, Lubrizol, 30% aktiv |
| 26 | Cetrimonium Chlorid | Dehyquart A-CA, BASF, 25% aktiv |
| 27 | Hydroxyethyl Cetyldimonium Phosphate | Luviquat Mono CP AT 1,BASF, 30% aktiv |
| 28 | Aminomethylpropanol | AMP Ultra PC 2000, Angus, 95% aktiv |
| 29 | Phenoxyethanol | Neolone PH 100 Preservative, Dow chemical, 1 00% aktiv |
| 30 | Parabene | Nipagin M, Clariant, 99% aktiv |
| 31 | Lanolin Alcohol | Super Hartolan B, Croda, 1 00% aktiv |
| 32 | Macadamia Integrifolia Seed Oil | Extrapone Macadamia Nut GW CL, Symrise, 3% aktiv |
| 33 | Polyquaternium-4 | Cellquat L-200, Akzo Nobel, 89% aktiv |

## Patentansprüche

1. Haar-Schaumfestigerzusammensetzungen enthaltend
- ein oder mehrere festigende Polymere, wobei das/die Polymer(e) Vinylpyrrolidone-Monomere enthalten,
- einen oder mehrere Cellulosemischether,
- einen oder mehrere Esterquat(s), wobei der/die Esterquat(s) eine Propylamin-Struktureinheit aufweist/en und
- Wasser,
wobei das oder die festigenden Polymere enthaltend ein Vinylpyrrolidone Monomer ausgewählt werden aus der Gruppe Polyvinylpyrrolidone und VP/VA Coplymer.

2. Haar-Schaumfestigerzusammensetzungen nach Anspruch 1, **dadurch gekennzeichnet, dass** ein oder mehrere Cellulosemischether ausgewählt werden aus der Gruppe Hydroxypropylmethylcellulosen, Hydroxyethyl-methylcellulosen, Hydroxyethylethylcellulosen und Hydroxybutylmethylcellulosen.

3. Haar-Schaumfestigerzusammensetzungen nach Anspruch 2, **dadurch gekennzeichnet, dass** der Cellulosmischether Hydroxypropylmethylcellulose ist.

4. Haar-Schaumfestigerzusammensetzungen nach wenigstens einem der vorstehenden Ansprüche, **dadurch gekennzeichnet, dass** der oder die Cellulosemischether in Gehalten von 0,02 Gew.-% bis 2,0. Gew.-%, bevorzugt von 0,05 Gew.-% bis 1,0 Gew.-%, insbesondere bevorzugt von 0,1 Gew.-% bis 0,5 Gew.-%, bezogen auf das Gesamtgewicht der Haar-Schaumfestigerzusammensetzung und den Aktivgehalt, vorliegt.

5. Haar-Schaumfestigerzusammensetzungen nach wenigstens einem der vorstehenden Ansprüche, **dadurch gekennzeichnet, dass** der oder die Esterquats enthaltend eine Propylamin-Struktureinheit Monoesterquats sind, insbesondere ausgewählt aus der Gruppe Stearylamidopropyltrimmonium Chloride, Ricinoleamidopropyltrimonnium Chloride und Palmitamidopropyltrimonium Chloride.

6. Haar-Schaumfestigerzusammensetzungen nach wenigstens einem der vorstehenden Ansprüche, **dadurch gekennzeichnet, dass** der Esterquat enthaltend eine Propylamin-Struktureinheit Palmitamidopropyltrimonium Chloride ist.

7. Haar-Schaumfestigerzusammensetzungen nach wenigstens einem der vorstehenden Ansprüche, **dadurch gekennzeichnet, dass** der oder die Esterquats enthaltend eine Propylamin-Struktureinheit in Gehalten von 0,05 Gew.-% bis 5,0 Gew.-%, bevorzugt von 0,1 Gew.-% bis 2,0 Gew.-%, insbesondere bevorzugt von 0,2 Gew.-% bis 1,0 Gew.-%, bezogen auf das Gesamtgewicht der Haar-Schaumfestigerzusammensetzung und den Aktivgehalt, vorliegt.

8. Haar-Schaumfestigerzusammensetzungen nach wenigstens einem der vorstehenden Ansprüche, **dadurch gekennzeichnet, dass** das oder die festigenden Polymere enthaltend ein Vinylpyrrolidone Monomere in Gehalten von 0,5 Gew.-% bis 20,0. Gew.-%, bevorzugt von 1,0 Gew.-% bis 10,0 Gew.-%, insbesondere bevorzugt von 1,5 Gew.-% bis 8,0 Gew.-%, bezogen auf das Gesamtgewicht der Haar-Schaumfestigerzusammensetzung und den Aktivgehalt, vorliegen.

9. Haar-Schaumfestigerzusammensetzungen nach wenigstens einem der vorstehenden Ansprüche, **dadurch gekennzeichnet, dass** zusätzlich ein oder mehrere weitere festigende Polymere, insbesondere ausgewählt aus der Gruppe Acrylates/Octylacrylamide Copolymer, AMP-Acrylates/Allyl Methacrylates Copolymer, Acrylates/Hydroxyesters Acrylates Copolymer und Acrylates Copolymer, enthalten sind.

10. Haar-Schaumfestigerzusammensetzungen nach wenigstens einem der vorstehenden Ansprüche, **dadurch gekennzeichnet, dass** das oder die zusätzlichen festigenden Polymere in Gehalten von 0,2 Gew.-% bis 15,0 Gew.-%, bevorzugt von 0,5 Gew.-% bis 12,0 Gew.-%, insbesondere bevorzugt von 1,0 Gew.-% bis 10,0 Gew.-%, bezogen auf das Gesamtgewicht der Haar-Schaumfestigerzusammensetzung und den Aktivgehalt, vorliegen.

11. Haar-Schaumfestigerzusammensetzungen nach wenigstens einem der vorstehenden Ansprüche, **dadurch gekennzeichnet, dass** ein oder mehrere kationische Polymere, insbesondere ausgewählt aus der Gruppe Polyquaternium-4, Polyquaternium-16 und Polyquaternium-68, enthalten sind.

12. Haar-Schaumfestigerzusammensetzungen nach wenigstens einem der vorstehenden Ansprüche, **dadurch gekennzeichnet, dass** das oder die kationischen Polymere in Gehalten von 0,01 Gew.-% bis 10,0 Gew.-%, bevorzugt von 0,02 Gew.-% bis 5,0 Gew.-%, insbesondere bevorzugt von 0,04 Gew.-% bis 2,0 Gew.-%, bezogen auf das Gesamtgewicht der Haar-Schaumfestigerzusammensetzung und den Aktivgehalt, vorliegen.

13. Haar-Schaumfestigerzusammensetzungen nach wenigstens einem der vorstehenden Ansprüche, **dadurch gekennzeichnet, dass** ein oder mehrere Substanzen der Vitamine der B Gruppe und/oder des Vitamin B-Komplexes enthalten sind.

14. Haar-Schaumfestigerzusammensetzungen nach wenigstens einem der vorstehenden Ansprüche, **dadurch gekennzeichnet, dass** die Substanz(en) der Vitamine der B Gruppe und/oder des Vitamin B-Komplexes ausgewählt werden aus der Gruppe Thiamin (Vitamin B1), Riboflavin (Vitamin B2), Nicotinsäure (Vitamin B3), Nicotinsäureamid (Niacinamid),

15. Haar-Schaumfestigerzusammensetzungen nach wenigstens einem der vorstehenden Ansprüche, **dadurch gekennzeichnet, dass** die Substanz der Vitamine der B Gruppe und/oder des Vitamin B-Komplexes Panthenol ist.

16. Haar-Schaumfestigerzusammensetzungen nach wenigstens einem der vorstehenden Ansprüche, **dadurch gekennzeichnet, dass** die Substanz(en) der Vitamine der B Gruppe und/oder des Vitamin B-Komplexes in Gehalten von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht der Haar-Schaumfestigerzusammensetzung und den Aktivgehalt, in den erfindungsgemäßen Zusammensetzungen vorliegen.

17. Haar-Schaumfestigerprodukt bestehend aus einer Aerosolpackungseinheit und einer Gesamtzusammensetzung, wobei die Gesamtzusammensetzung eine Haar-Schaumfestigerzusammensetzung nach Anspruch 1 und ein Treibmittel ist.

18. Haar-Schaumfestigerprodukt nach Anspruch 17, **dadurch gekennzeichnet, dass** das Treibmittel ein oder mehrere Treibgase ausgewählt aus der Gruppe Kohlenwasserstoffgasen, fluorierten Gasen, Fluorkohlenwasserstoffgase, Dimethylether, Stickstoff, Luft oder Kohlendioxid oder Mischungen davon, umfasst.

19. Haar-Schaumfestigerprodukt nach Anspruch 18, **dadurch gekennzeichnet, dass** das Treibmittel eine Mischung aus den Treibgasen Isobutan, Propan und n-Butan ist.

20. Haar-Schaumfestigerprodukt nach wenigstens einem der Ansprüche 17 bis 19, **dadurch gekennzeichnet, dass** das Treibmittel eine Druckgasstufe ausgewählt aus dem Bereich von 2,7 bis 3,5 bar aufweist.

21. Haar-Schaumfestigerprodukt nach wenigstens einem der Ansprüche 17 bis 20, **dadurch gekennzeichnet, dass** der Gewichtsanteil an Treibmittel aus dem Bereich von 1,0 bis 15 Gew.-%, insbesondere 2,0 bis 12,0 Gew.-% gewählt wird, bezogen auf das Gesamtgewicht der Gesamtzusammensetzung.

22. Haar-Schaumfestigerprodukt nach wenigstens einem der Ansprüche 17 bis 21, **dadurch gekennzeichnet, dass** die Schaumfestigerzusammensetzungen in Form von verschäumbaren oder nachschäumbaren Zusammensetzungen vorliegen.

## Claims

1. Hair mousse compositions comprising
- one or more setting polymers, wherein the polymer(s) comprise vinylpyrrolidone monomers,
- one or more cellulose mixed ethers,
- one or more ester quats(s), wherein the ester quat(s) has/have a propylamine structural unit and
- water,
wherein the setting polymer(s) comprising a vinylpyrrolidone monomer are selected from the group of polyvinylpyrrolidones and VP/VA copolymer.

2. Hair mousse compositions according to Claim 1, **characterized in that** one or more cellulose mixed ethers are selected from the group comprising hydroxypropyl methylcelluloses, hydroxyethyl methylcelluloses, hydroxyethyl ethylcelluloses and hydroxybutyl methylcelluloses.

3. Hair mousse compositions according to Claim 2, **characterized in that** the cellulose mixed ether is hydroxypropyl methylcellulose.

4. Hair mousse compositions according to at least one of the preceding claims, **characterized in that** the cellulose mixed ether(s) is present at contents of 0.02% by weight to 2.0% by weight, preferably 0.05% by weight to 1.0% by weight, particularly preferably 0.1% by weight to 0.5% by weight, based on the total weight of the hair mousse composition and the active content.

5. Hair mousse compositions according to at least one of the preceding claims, **characterized in that** the ester quat(s) comprising one propylamine structural unit are monoester quats, particularly selected from the group consisting of stearylamidopropyltrimonium chloride, ricinoleamidopropyltrimonium chloride and palmitamidopropyltrimonium chloride.

6. Hair mousse compositions according to at least one of the preceding claims, **characterized in that** the ester quat comprising a propylamine structural unit is palmitamidopropyltrimonium chloride.

7. Hair mousse compositions according to at least one of the preceding claims, **characterized in that** the ester quat(s) comprising a propylamine structural unit is present at contents of 0.05% by weight to 5.0% by weight, preferably 0.1% by weight to 2.0% by weight, particularly preferably 0.2% by weight to 1.0% by weight, based on the total weight of the hair mousse composition and the active content.

8. Hair mousse compositions according to at least one of the preceding claims, **characterized in that** the setting polymer(s) comprising a vinylpyrrolidone monomer are present at contents of 0.5% by weight to 20.0% by weight, preferably 1.0% by weight to 10.0% by weight, particularly preferably 1.5% by weight to 8.0% by weight, based on the total weight of the hair mousse composition and the active content.

9. Hair mousse compositions according to at least one of the preceding claims, **characterized in that** in addition one or more further setting polymers are present, in particular selected from the group comprising acrylates/octylacrylamide copolymer, AMP-acrylates/allyl methacrylates copolymer, acrylates/hydroxyesters acrylates copolymer and acrylates copolymer.

10. Hair mousse compositions according to at least one of the preceding claims, **characterized in that** the additional setting polymer(s) are present at contents of 0.2% by weight to 15.0% by weight, preferably 0.5% by weight to 12.0% by weight, particularly preferably 1.0% by weight to 10.0% by weight, based on the total weight of the hair mousse composition and the active content.

11. Hair mousse compositions according to at least one of the preceding claims, **characterized in that** one or more cationic polymers are present, in particular selected from the group consisting of polyquaternium-4, polyquaternium-16 and polyquaternium-68.

12. Hair mousse compositions according to at least one of the preceding claims, **characterized in that** the cationic polymer(s) are present at contents of 0.01% to 10.0% by weight, preferably 0.02% by weight to 5.0% by weight, particularly preferably 0.04 to 2.0% by weight, based on the total weight of the hair mousse composition and the active content.

13. Hair mousse compositions according to at least one of the preceding claims, **characterized in that** one or more substances of the vitamins of the B group and/or of the vitamin B complexes are present.

14. Hair mousse compositions according to at least one of the preceding claims, **characterized in that** the substance(s) of the vitamins of the B group and/or of the vitamin B complexes are selected from the group comprising thiamine (vitamin B1), riboflavin (vitamin B2), nicotinic acid (vitamin B3), nicotinamide (niacinamide).

15. Hair mousse compositions according to at least one of the preceding claims, **characterized in that** the substance of the vitamins of the B group and/or of the vitamin B complexes is panthenol.

16. Hair mousse compositions according to at least one of the preceding claims, **characterized in that** the substance(s) of the vitamins of the B group and/or of the vitamin B complexes are present in the compositions according to the invention at contents of 0.005% by weight to 5% by weight, preferably 0.05% by weight to 2% by weight, particularly preferably 0.01 to 1.0% by weight, based on the total weight of the hair mousse composition and the active content.

17. Hair mousse product consisting of an aerosol packaging unit and a total composition, wherein the total composition is a hair mousse composition according to Claim 1 and a propellant.

18. Hair mousse product according to Claim 17, **characterized in that** the propellant comprises one or more propellant gases selected from the group consisting of hydrocarbon gases, fluorinated gases, hydrofluorocarbon gases, dimethyl ether, nitrogen, air or carbon dioxide or mixtures thereof.

19. Hair mousse product according to Claim 18, **characterized in that** the propellant gas is a mixture of the propellant gases isobutane, propane and n-butane.

20. Hair mousse product according to at least one of Claims 17 to 19, **characterized in that** the propellant has a gas pressure rating selected from the range from 2.7 to 3.5 bar.

21. Hair mousse product according to at least one of Claims 17 to 20, **characterized in that** the proportion by weight of propellant is selected from the range of 1.0 to 15% by weight, particularly 2.0 to 12.0% by weight, based on the total weight of the total composition.

22. Hair mousse product according to at least one of Claims 17 to 21, **characterized in that** the mousse compositions are in the form of foamable or post-foamable compositions.

## Revendications

1. Compositions de fixateur en mousse pour cheveux contenant
- un ou plusieurs polymères fixants, le(s) polymère(s) contenant des monomères de vinylpyrrolidone,
- un ou plusieurs éthers mixtes de cellulose,
- un ou plusieurs esterquat(s), l'esterquat ou les esterquats présentant un motif structural de propylamine et
- de l'eau,
le(s) polymère(s) contenant un monomère de vinylpyrrolidone étant choisi(s) dans le groupe de la polyvinylpyrrolidone et d'un copolymère VP/VA.

2. Compositions de fixateur en mousse pour cheveux selon la revendication 1, **caractérisées en ce qu'**un ou plusieurs éthers mixtes de cellulose sont choisis dans le groupe des hydroxypropylméthylcelluloses, des hydroxyéthyl-méthylcelluloses, des hydroxyéthyl-éthylcelluloses et des hydroxybutylméthylcelluloses.

3. Compositions de fixateur en mousse pour cheveux selon la revendication 2, **caractérisées en ce que** l'éther mixte de cellulose est l'hydroxypropylméthylcellulose.

4. Compositions de fixateur en mousse pour cheveux selon au moins l'une des revendications précédentes, **caractérisées en ce que** l'éther mixte de cellulose est présent en des teneurs de 0,02 % en poids à 2,0 % en poids, préférablement de 0,05 % en poids à 1,0 % en poids, en particulier préférablement de 0,1 % en poids à 0,5 % en poids, par rapport au poids total de la composition de fixateur en mousse pour cheveux et au contenu actif.

5. Compositions de fixateur en mousse pour cheveux selon au moins l'une des revendications précédentes, **caractérisées en ce que** l'esterquat ou les esterquats contenant un motif structural de propylamine sont des monoesterquats, en particulier choisis dans le groupe du chlorure de stéarylamidopropyltrimmonium, du chlorure de ricinoléamido-propyltrimmonium et du chlorure de palmitamidopropyltrimmonium.

6. Compositions de fixateur en mousse pour cheveux selon au moins l'une des revendications précédentes, **caractérisées en ce que** l'esterquat contenant un motif structural de propylamine est le chlorure de palmitamidopropyltrimmonium.

7. Compositions de fixateur en mousse pour cheveux selon au moins l'une des revendications précédentes, **caractérisées en ce que** l'esterquat ou les esterquats contenant un motif structural de propylamine sont présents en des teneurs de 0,05 % en poids à 5,0 % en poids, préférablement de 0,1 % en poids à 2,0 % en poids, en particulier préférablement de 0,2 % en poids à 1,0 % en poids, par rapport au poids total de la composition de fixateur en mousse pour cheveux et au contenu actif.

8. Compositions de fixateur en mousse pour cheveux selon au moins l'une des revendications précédentes, **caractérisées en ce que** les polymères fixants contenant un monomère de vinylpyrrolidone sont présents en des teneurs de 0,5 % en poids à 20,0 % en poids, préférablement de 1,0 % en poids à 10,0 % en poids, en particulier préférablement de 1,5 % en poids à 8,0 % en poids, par rapport au poids total de la composition de fixateur en mousse pour cheveux et au contenu actif.

9. Compositions de fixateur en mousse pour cheveux selon au moins l'une des revendications précédentes, **caractérisées en ce que**, de plus, un ou plusieurs polymères fixants supplémentaires, en particulier choisis dans le groupe d'un copolymère acrylate/octylacrylamide, d'un copolymère AMP-acrylate/méthacrylate d'allyle, d'un copolymère acrylate/hydroxyester d'acrylate et d'un copolymère d'acrylate, sont contenus.

10. Compositions de fixateur en mousse pour cheveux selon au moins l'une des revendications précédentes, **caractérisées en ce que** le(s) polymère(s) fixant(s) supplémentaire(s) sont présents en des teneurs de 0,2 % en poids à 15,0 % en poids, préférablement de 0,5 % en poids à 12,0 % en poids, en particulier préférablement de 1,0 % en poids à 10,0 % en poids, par rapport au poids total de la composition de fixateur en mousse pour cheveux et au contenu actif.

11. Compositions de fixateur en mousse pour cheveux selon au moins l'une des revendications précédentes, **caractérisées en ce qu'**un ou plusieurs polymères cationiques, en particulier choisis dans le groupe du polyquaternium-4, du polyquaternium-16 et du polyquaternium-68, sont contenus.

12. Compositions de fixateur en mousse pour cheveux selon au moins l'une des revendications précédentes, **caractérisées en ce que** le(s) polymère(s) cationique(s) sont présents en des teneurs de 0,01 % en poids à 10,0 % en poids, préférablement de 0,02 % en poids à 5,0 % en poids, en particulier préférablement de 0,04 % en poids à 2,0 % en poids, par rapport au poids total de la composition de fixateur en mousse pour cheveux et au contenu actif.

13. Compositions de fixateur en mousse pour cheveux selon au moins l'une des revendications précédentes, **caractérisées en ce qu'**une ou plusieurs substances des vitamines du groupe B et/ou du complexe de vitamine B sont contenues.

14. Compositions de fixateur en mousse pour cheveux selon au moins l'une des revendications précédentes, **caractérisées en ce que** le(s) substance(s) des vitamines du groupe B et/ou du complexe de vitamine B sont choisies dans le groupe de la thiamine (vitamine B1), de la riboflavine (vitamine B2), de l'acide nicotinique (vitamine B3), du nicotinamide (niacinamide).

15. Compositions de fixateur en mousse pour cheveux selon au moins l'une des revendications précédentes, **caractérisées en ce que** la substance des vitamines du groupe B et/ou du complexe de vitamine B est le panthénol.

16. Compositions de fixateur en mousse pour cheveux selon au moins l'une des revendications précédentes, **caractérisées en ce que** la ou les substance(s) des vitamines du groupe B et/ou du complexe de vitamine B sont présentes en des teneurs de 0,005 % en poids à 5 % en poids, préférablement de 0,05 % en poids à 2 % en poids, en particulier préférablement de 0,01 % en poids à 1,0 % en poids, par rapport au poids total de la composition de fixateur en mousse pour cheveux et au contenu actif, dans les compositions selon l'invention.

17. Produit de fixateur en mousse pour cheveux composé d'une unité d'emballage d'aérosol et d'une composition totale, la composition totale étant une composition de fixateur en mousse pour cheveux selon la revendication 1 et un propulseur.

18. Produit de fixateur en mousse pour cheveux selon la revendication 17, **caractérisé en ce que** le propulseur comprend un ou plusieurs gaz propulseurs choisis dans le groupe des gaz d'hydrocarbure, des gaz fluorés, des gaz de fluorohydrocarbure, du diméthyléther, de l'azote, de l'air et du dioxyde de carbone et des mélanges correspondants.

19. Produit de fixateur en mousse pour cheveux selon la revendication 18, **caractérisé en ce que** le propulseur est un mélange composé des gaz propulseurs isobutane, propane et n-butane.

20. Produit de fixateur en mousse pour cheveux selon au moins l'une des revendications 17 à 19, **caractérisé en ce que** le propulseur présente un étage de gaz comprimé choisi dans la plage de 2,7 à 3,5 bars.

21. Produit de fixateur en mousse pour cheveux selon au moins l'une des revendications 17 à 20, **caractérisé en ce que** la proportion en poids de propulseur est choisie dans la plage de 1,0 à 15 % en poids, en particulier de 2,0 à 12,0 % en poids, par rapport au poids total de la composition totale.

22. Produit de fixateur en mousse pour cheveux selon au moins l'une des revendications 17 à 21, **caractérisé en ce que** les compositions de fixateur en mousse sont présentes sous forme de compositions moussables ou moussables a posteriori.
